# EUROPEAN PATENT APPLICATION

(11) **EP 2 832 358 A1**
(43) Date of publication of application: **04.02.2015**
(21) Application number: 13179049.5
(22) Date of filing: 02.08.2013
(51) Int. Cl.: A61K 31/513, A61K 31/519, A61P 35/00

(54) **Pharmaceutical kit for use in the treatment of colon and colorectal cancer**

(71) Applicant: Bionsil S.r.l., 20123 Milano (IT)
(72) Inventor: Lavitrano, Marialuisa, 20129 MILANO (IT); Grassilli, Emanuela, 20900 MONZA (IT); Giovannoni, Roberto, 20161 MILANO (IT); Pisano, Fabio, 89040 MONSTERACE (IT); Romano, Gabriele, 21010 GERMIGNAGA (IT); Masiero, Laura, 20091 BRESSO (IT); Cerrito, Maria Grazia, 20131 MILANO (IT)
(74) Representative: Zaccaro, Elisabetta

(57) **Abstract**

The present invention describes therapies for the effective treatment of colon and colorectal carcinomas. The present invention relates to a pharmaceutical kit comprising ibrutinib and fluorouracil, for the treatment of colon and colorectal carcinomas also in the case in which such carcinomas are drug resistant and therefore allows to overcome cancer drug resistance.

## Description

### Field of the invention

The present invention describes novel therapies for the effective treatment of drug resistant colon and colorectal carcinomas.

### State of the art

Apoptosis is a common mode of eukaryotic cell death that is triggered by an inducible cascade of biochemical events leading to the activation of DNA cleavage. Most chemotherapeutic agents exert their anticancer activity by inducing apoptosis. Cell resistance to apoptosis is therefore a major factor which limits the effective anticancer therapy.

Bruton's tyrosine kinase (BTK), a member of the Tec family of cytoplasmic tyrosine kinases, is intimately involved in multiple signal-transduction pathways regulating survival, activation, proliferation, and differentiation of B-lineage lymphoid cells.

BTK has been reported responsible for triggering radiation-induced apoptosis of lymphoma B cells, and its kinase domain is indispensable for the apoptotic response. BTK and other members of the Tec kinase family are expressed in various tissues, and recent studies suggest that since BTK is an apoptosis modulating molecule, alteration of its function could be cause of various types of cancer.

WO2008110624 describes the identification and isolation of a novel isoform of the BTK protein, having a molecular weight of 65-68 kDa. The novel BTK protein, also identified as p65BTK, is shorter than the BTK identified in B cells, and its mRNA has a different first exon.

p65BTK was identified in epithelial carcinoma cell lines such as breast, ovary, lung and colon. In particular p65BTK was seen to have a high expression in epithelial carcinoma cells which were proved resistant to chemotherapeutic agents.

In WO2008110624 it is shown that resistance to chemotherapeutic drugs can be reverted by inhibiting p65BTK expression, indicating the achievement of a first step to cancer treatment.

WO2008039218 describes compounds that inhibit BTK, by forming a covalent bond with a cysteine residue of BTK. One of the compounds described is 1-(3-(4-amino-3-(4-phenoxyphenyl)-1H-pyrazolo[3,4-d]pyrimidin-1-yl)piperidin-1-yl)prop-2-en-1-one (Compound 4).

WO2010009342 describes BTK inhibitor compounds such as 1-(3-(4-amino-3-(4-phenoxyphenyl)-1H-pyrazolo[3,4-d]pyrimidin-1-yl)piperidin-1-yl)prop-2-en-1-one, and their use in the treatment of tumors. Among the therapeutic uses of the inhibitor compounds, WO2010009342 indicates the inhibition of lymphoma tumor cell growth, the inhibition of collagen-induced arthritis, the inhibition of lupus and of mast cell degeneration. WO2010009342 also indicates the administration of a BTK inhibitor (Compound 1) to a mouse model for colon cancer. A colon cancer xenograft is implanted under the skin of a mouse and the size of the tumor xenograft is monitored upon Compound 1 administration.

Many cancers develop resistance to chemotherapy drugs, a major factor in the failure of many forms of chemotherapy. Drug resistance affects patients with a variety of blood cancers and solid tumors, including breast, ovarian, lung, and lower gastrointestinal tract cancers.

The need and importance is increasingly felt for novel therapies for the effective treatment of colon and colorectal carcinomas, and drug-resistant colon and colorectal carcinomas once the resistance to chemotherapeutic drugs is acknowledged.

It is therefore object of the present invention the effective treatment of colon and colorectal carcinomas, both drug-sensitive and drug-resistant.

### Summary of the invention

The present inventors have surprisingly found that if you administer ibrutinib and fluorouracil to colon carcinomas and to colorectal carcinomas, said carcinomas are treated.

Therefore the present invention concerns a pharmaceutical kit for use in the treatment of colon and colorectal carcinomas.

In particular the invention relates to a pharmaceutical kit for simultaneous, sequential and separate use of an effective dose of ibrutinib and an effective dose of fluorouracil, in the treatment of colon and colorectal carcinomas, both drug-sensitive and drug-resistant.

The invention further relates to a method for the treatment of resistant colon and colorectal carcinomas, comprising the administration of and effective dose of ibrutinib and of an effective dose of fluorouracil.

As will be further described in the detailed description of the invention, the pharmaceutical kit of the present invention has the advantages of being specific for the treatment of colon and colorectal carcinomas, both drug-sensitive and drug-resistant.

### Brief description of the drawings

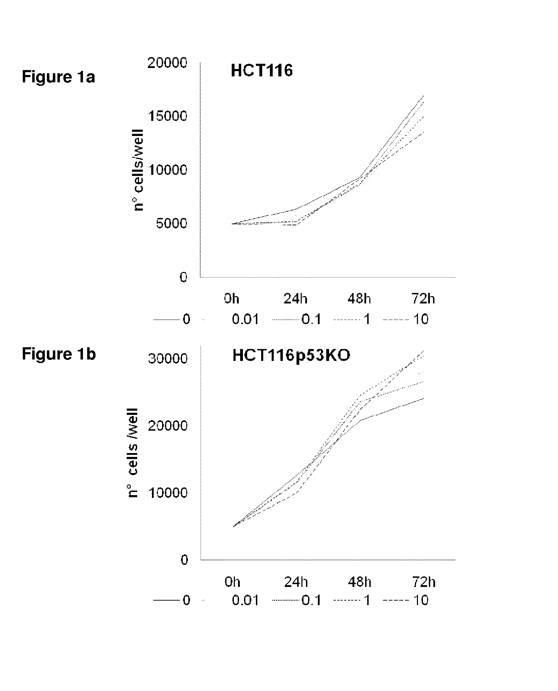

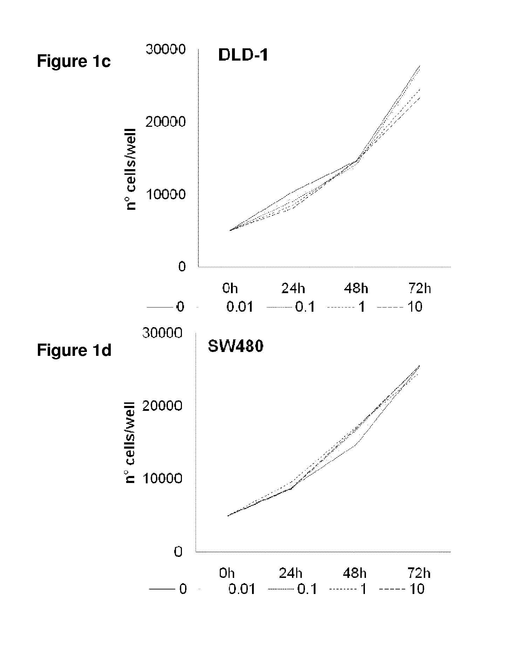

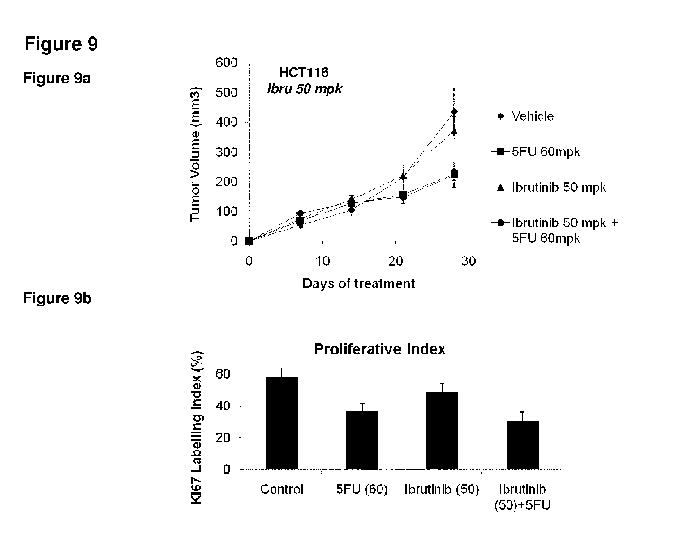

The characteristics and advantages of the present invention will be apparent from the detailed description reported below, from the Examples given for illustrative and nonlimiting purposes, and from the annexed Figures 1-10, wherein:
Figure 1 shows the effect of different concentrations of Ibrutinib on cell proliferation as illustrated in particular in the growth curves (short term assays) as described in Example 1:
   Figure 1 a: dose-response curve of HCT116 cells grown in presence of increasing concentrations of ibrutinib (from0 to 10 µM), for 72 hours. ; 5000 cells/well were seeded in triplicates and cell number was assessed each 24 hs by use of CellTiter 96 AQueous Non-Radioactive Cell Proliferation Assay (Promega).
   Figure 1b: dose-response curve of HCT116p53KO cells grown in presence of increasing concentrations of ibrutinib (from 0 to 10 µM) for 72 hours ; 5000 cells/well were seeded in triplicates and cell number was assessed each 24 hs by use of CellTiter 96 AQueous Non-Radioactive Cell Proliferation Assay (Promega).
   Figure 1c: dose-response curve of DLD-1 cells grown in presence of increasing concentrations of ibrutinib (from 0 to 10 µM) for 72 hours; 5000 cells/well were seeded in triplicates and cell number was assessed each 24 hs by use of CellTiter 96 AQueous Non-Radioactive Cell Proliferation Assay (Promega).
   Figure 1d: dose-response curve of SW480 cells grown in presence of increasing concentrations of ibrutinib (from 0 to 10 µM) for 72 hours; 5000 cells/well were seeded in triplicates and cell number was assessed each 24 hs by use of CellTiter 96 AQueous Non-Radioactive Cell Proliferation Assay (Promega).

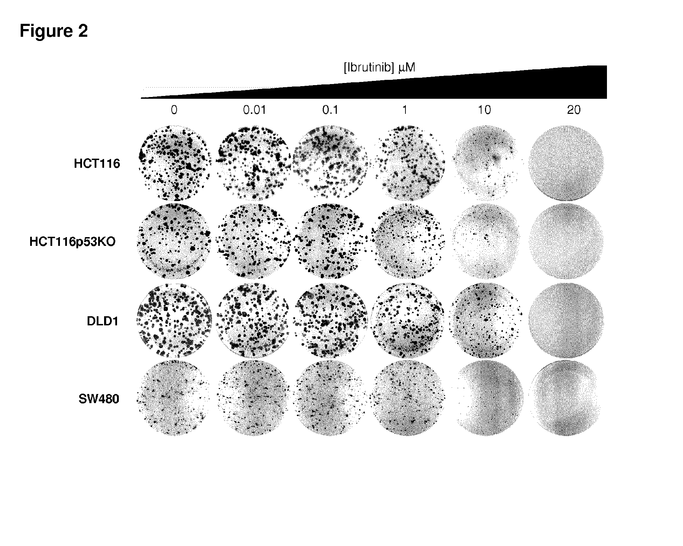

Figure 2 shows the effect of different concentrations of Ibrutinib (from 0 to 20 µM) on the proliferation of three colon carcinoma cell lines which are resistant to 5FU (HCT116p53KO, SW480 and DLD-1) and on the sensitive HCT116 cell line as illustrated in the colony assay experiment (long term assay) described in Example 2.

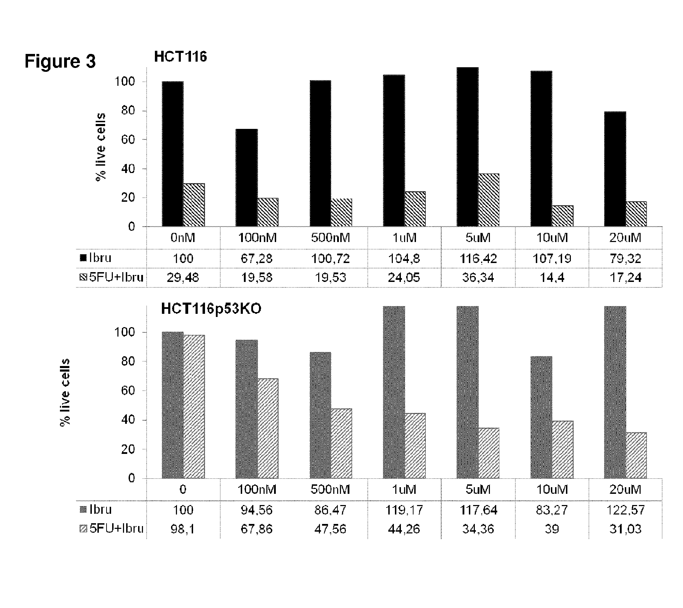

Figure 3 shows the effect of different concentrations of Ibrutinib (from 0 to 20 µM) and of Ibrutinib (from 0 to 20 µM) + 5FU (200 µM ) on viability of HCT116 and HCT116p53KO colon carcinoma cell lines as described in Example 3.

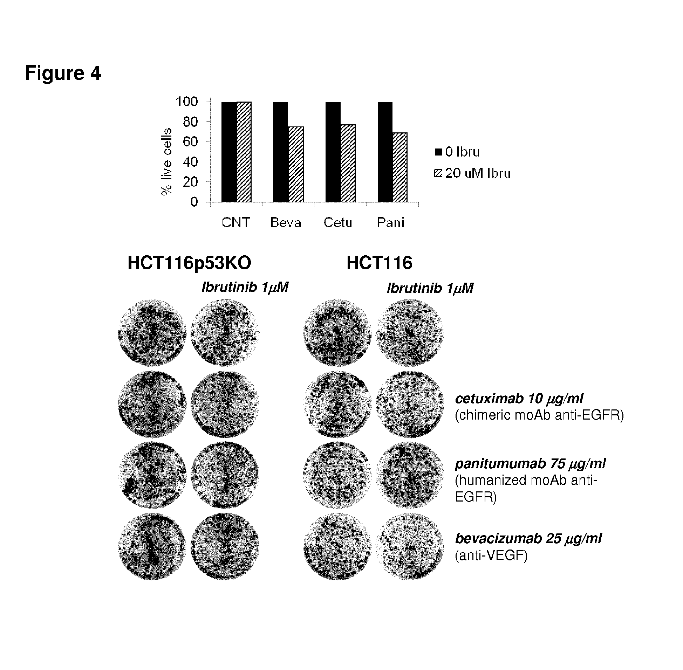

Figure 4 shows the results of the combined treatment of ibrutinib and three different anti-tumour ( "targeted" drugs for the inhibition of EGFR (cetuximab, panitumumab) and of VEGF (bevacizumab) as described in Example 4.

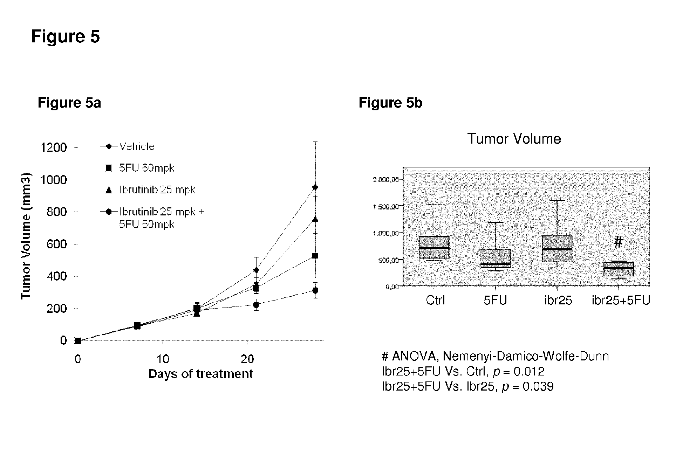

Figure 5a shows the growth curve of tumors derived from HCT116p53KO cells xenografted in CD1 mice after treatment with Ibrutinib (25mpk), 5FU (60mpk) and Ibrutinib (25mpk) + 5FU (60mpk), compared to the control (vehicle) as described in Example 5.
Figure 5b shows the range of tumor volumes measured at the end of the treatment of the mice xenografted with HCT116p53KO cellswith Ibrutinib (25mpk), 5FU (60mpk), Ibrutinib (25mpk) + 5FU (60mpk), and vehicle alone (ctrl) . In the box plot graph of
Figure 5b, the bold line indicates the median of the values, the box indicates the first and third quartile. The maximum and minimum values for each group are also reported as described in Example 5
Figure 6a shows the growth curve of tumors derived from HCT116p53KO cells xenografted in CD1 mice after treatment with Ibrutinib (50mpk), 5FU (60mpk) and Ibrutinib (50mpk) + 5FU (60mpk), compared to the control (vehicle) as described in Example 5.
Figure 6b shows the range of tumor volumes measured at the end of the treatment of the mice xenografted with HCT116p53KO cells with Ibrutinib (50mpk), 5FU (60mpk), Ibrutinib (50mpk) + 5FU (60mpk), and vehicle alone (ctrl) . In the box plot graph of

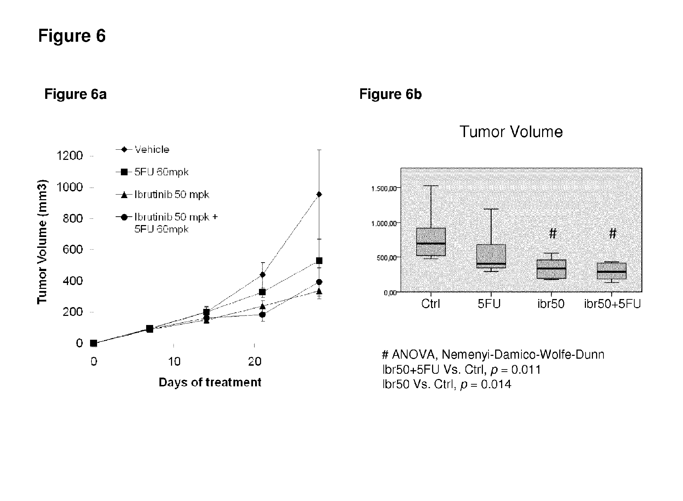

Figure 6b, the bold line indicates the median of the values, the box indicates the first and third quartile. The maximum and minimum values for each group are also reported as described in Example 5.

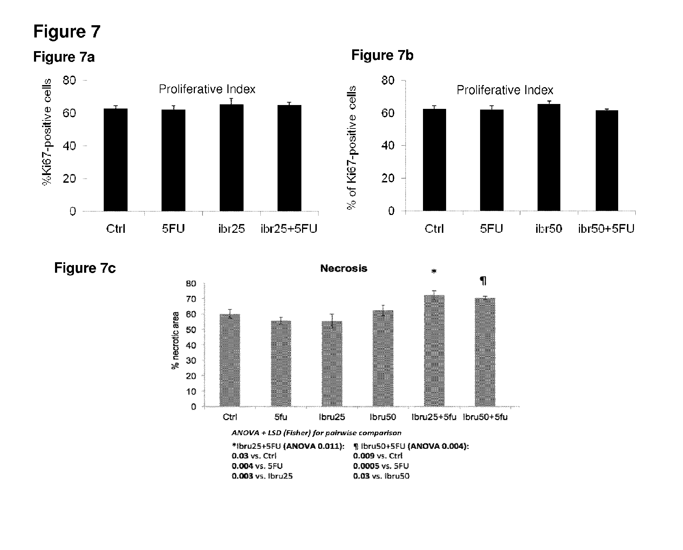

Figure 7a shows the proliferative index, evaluated as the percentage of Ki67-positive cells, of the tumoral tissues excised from the mice at the end of the treatment at the end of the treatment with vehicle alone (Ctrl), 5FU (60mpk), Ibrutinib 25 mpk, 5FU (60mpk) + Ibrutinib 25mpk. Data are expressed as mean ± SEM.
Figure 7b shows the proliferative index, evaluated as the percentage of Ki67-positive cells, of the tumoral tissues excised from the mice at the end of the treatment at the end of the treatment with vehicle alone (Ctrl), 5FU (60mpk), Ibrutinib 50 mpk, 5FU (60mpk) + Ibrutinib 50mpk. Data are expressed as mean ± SEM.
Figure 7c shows the percentage of necrotic area measured in the tumoral tissues excised from the mice at the end of the treatment with vehicle alone (Ctrl), 5FU (60mpk), Ibrutinib 25 mpk, Ibrutinib 50 mpk, 5FU (60mpk) + Ibrutinib 25mpk, 5FU (60mpk) + Ibrutinib 50mpk. Data are expressed as mean ± SEM as described in Example 5.

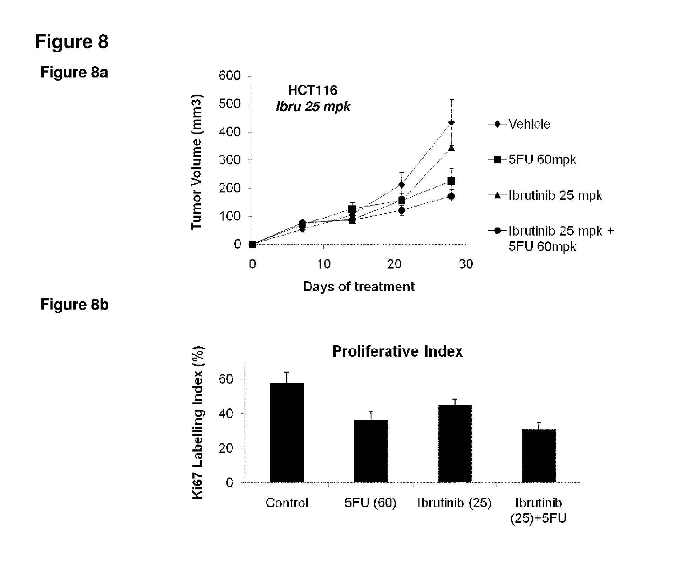

Figure 8a shows the growth curve of tumors derived from HCT116 xenografted in CD1 mice after treatment with Ibrutinib (25mpk), 5FU (60mpk) and Ibrutinib (25mpk) + 5FU (60mpk), compared to the control (vehicle)
The proliferative index of the tumoral tissues excised from the mice at the end of the treatment, evaluated as the percentage of Ki67-positive cells, is shown in Figure 8b.
Data are expressed as mean ± SEM as described in Example 5 Figure 9a shows the growth curve of tumors derived from HCT116 xenografted in CD1 mice after treatment of with Ibrutinib (50mpk), 5FU (60mpk) and Ibrutinib (50mpk) + 5FU (60mpk), compared to the control (vehicle)).
Figure 9b shows the proliferative index of the tumoral tissues excised from the mice at the end of the treatment evaluated as the percentage of Ki67-positive cells. Data is expressed as mean ± SEM as described in Example 5.

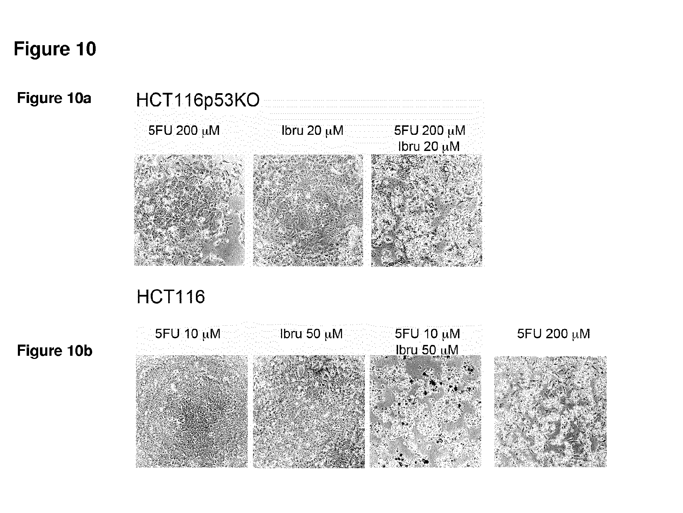

Figure 10 shows the effect of combining 5FU and Ibrutinib treatments in drug-resistant (HCT116p53KO) and -sensitive (HCT116) colon carcinoma cells. In Figure 10a are shown representative photographs of HCT116p53KO cells taken at 72 hours of incubation in presence of 200 µM 5FU, 20 µM Ibrutinib, 200 µM 5FU + 20 µM Ibrutinib.
Figure 10b shows representative photographs of HCT116 cells taken at 72 hours of incubation in presence of 200 µM 5FU, 10 µM 5FU, 50 µM Ibrutinib, 10 µM 5FU + 50 µM Ibrutinib as as described in Example 6.

### Detailed description of the invention

The present invention concerns a pharmaceutical kit for simultaneous, sequential and separate use of an effective dose of ibrutinib and an effective dose of fluorouracil, in the treatment of colon and colorectal carcinomas.

The inventors have surprisingly found that the treatment of colon or colorectal carcinomas by the simultaneous or sequential administration of ibrutinib and fluorouracil results in a synergic antitumoral effect compared to the administration of either of the components individually. This surprising synergic antitumoral effect has been seen also in drug-resistant colon and colorectal carcinomas, which are those cancers which develop resistance to chemotherapy drugs. The pharmaceutical kit according to the present invention allows to overcome cancer drug resistance due to the many mechanisms of tumor cell evolution and adaptation.

A further surprising advantage of the present invention relates to the fact that the inventors have found that in the treatment of colon or colorectal carcinomas by the simultaneous or sequential administration of ibrutinib and fluorouracil, a smaller or lower amount of the chemotherapeutic drug can be used and is efficacious, in comparison to the amount of chemotherapeutic drug which is used in the treatment of the same carcinomas.

The amount of chemotherapeutic drug, fluorouracil, can be reduced in a range from 5% to 80%, preferably in a range from 10% to 60%, more preferably from 20% to 40%, still more preferably from 25% to 35%.

The present invention further concerns a pharmaceutical kit for simultaneous, sequential and separate use of an effective dose of ibrutinib and an effective dose of fluorouracil, in the treatment of drug-resistant colon and colorectal carcinomas.

In the present invention, by Fluorouracil or 5-FU is intended an antimetabolite drug used in the treatment of cancer, which acts through the irreversible inhibition of thymidylate synthase. 5-FU induces cell cycle arrest and apoptosis by inhibiting the cell's ability to synthesize DNA. 5-FU has the following IUPAC name: 5-fluoro-1H,3H-pyrimidine-2,4-dione, and CAS number: 51-21-8.

In the present invention, by Ibrutinib or ibru is intended a compound of formula 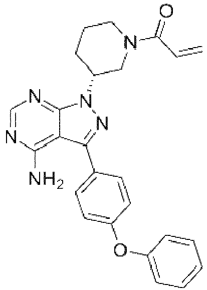 with the CAS identifier number: 936563-96-1, and chemical IUPAC name: 1-[(3R)-3-[4-amino-3-(4-phenoxyphenyl)pyrazolo[3,4-d]pyrimidin-1-yl]piperidin-1-yl]prop-2-en-1-one.

Ibrutinib has been used in preclinical studies to for the treatment of chronic lymphocytic leukemia (CLL), and has been shown to promote apoptosis, inhibit proliferation, and also prevent CLL cells from responding to survival stimuli provided by the microenvironment. Ibrutinib is a selective and irreversible inhibitor of the enzyme Bruton tyrosine kinase (Btk), that forms a covalent bond with a cysteine residue on Btk.

Preclinical studies suggest that the treatment with ibrutinib has a safe toxicological profile, thus a benefit to cancer patients who usually experience the adverse side effects of chemotherapy such as fatigue, loss of appetite, nausea or vomiting.

In a second embodiment the pharmaceutical kit of the present invention surprisingly allows the treatment of drug-resistant cancer cells in a way that not only shows a synergic antitumoral effect of ibrutinib and fluorouracil, but also allow the use of lower amounts of fluorouracil, and thus a lower effective dose of the chemotheurapeutic drug, thus avoiding the undesirable and often harmful side-effects that these drugs have.

A further aspect of the present invention is a pharmaceutical kit for simultaneous, sequential and separate use of an effective dose of ibrutinib and an effective dose of fluorouracil, wherein the ibrutinib is in the form of a pharmaceutically acceptable salt. In a further aspect, the invention provides a pharmaceutical kit comprising an effective dose of ibrutinib and an effective dose of fluorouracil, wherein the effective dose of ibrutinib and the effective dose of 5-fluorouracil are formulated as a pharmaceutical form selected from oral form, parenteral form and rectal form.

Depending on the method/route of administration, dosage forms can be of several types. These include many kinds of liquid, solid, and semisolid dosage forms. Common dosage forms include pill, tablet, or capsule, drink or syrup. The route of administration (ROA) for drug delivery is dependent on the dosage form of the substance.

Various dosage forms may exist for a single particular drug, since different medical conditions can warrant different routes of administration.

Routes of administration can be inhalational, buccal (oral), sublingual, nasal, suppository and parenteral.

In a still further aspect, the invention provides a pharmaceutical kit comprising an effective dose of ibrutinib and an effective dose of fluorouracil, wherein the pharmaceutical form is an oral form, preferably a tablet.

Furthermore, the invention relates to a pharmaceutical kit for simultaneous, sequential and separate use of an effective dose of ibrutinib and an effective dose of fluorouracil, wherein the pharmaceutical form comprises at least one pharmaceutical acceptable excipient.

An excipient is a pharmacologically inactive substance formulated with the active ingredient, commonly used to bulk up formulations that contain potent active ingredients (often referred to as "bulking agents," "fillers," or "diluents"), to allow convenient and accurate dispensation of a drug substance when producing a dosage form. They also can serve various therapeutic-enhancing purposes, such as facilitating drug absorption or solubility, or other pharmacokinetic considerations.

The selection of appropriate excipients also depends upon the route of administration and the dosage form, as well as the active ingredient and other factors.

In a preferred embodiment, the invention provides a pharmaceutical kit comprising an effective dose of ibrutinib and an effective dose of fluorouracil, wherein the effective dose of ibrutinib is in the range of from 1 mg/day to 2000 mg/day (derived from ClinicalTrials.gov; Study Identifiers: NCT01804686, NCT01589302, NCT01855750, NCT01744691, NCT01105247, NCT00849654) and wherein the effective dose of fluorouracil is in the range of from 400 mg/m2 to 600 mg/m2 (derived from the NCI page for colon cancer treatment for heath professionals http://www.cancer.qov/cancertopicslpdq/treatment/colon/HealthProfessional/page8).

In a more preferred embodiment the invention provides a pharmaceutical kit wherein the effective dose of ibrutinib is in the range of from 10 mg/day to 1100 mg/day.

In a more preferred embodiment the invention provides a pharmaceutical kit wherein the effective dose of ibrutinib is in the range of from 20 mg/day to 950 mg/day.

In a still more preferred embodiment, the invention provides a pharmaceutical kit comprising an effective dose of ibrutinib and an effective dose of fluorouracil, wherein the effective dose of ibrutinib is in the range of from 25 mg/day to 840 mg/day.

In a preferred embodiment the invention provides a pharmaceutical kit wherein the effective dose of fluorouracil is in the range of from 450 mg/m² to 550 mg/m².

In a still preferred embodiment the invention provides a pharmaceutical kit wherein the effective dose of fluorouracil is lower, preferably in a range from 150 mg/m² to 400 mg/m²_{.}

In a further aspect of the present invention the pharmaceutical kit comprises at least one container comprising the effective dose of ibrutinib and at least one container comprising the effective dose of fluorouracil, and an instruction leaflet.

In a still further aspect of the present invention provides the use of an effective dose of ibrutinib and an effective dose of fluorouracil, in the treatment of colon and colorectal carcinomas.

The use according to the present invention advantageously allows for the treatment of colon and colorectal carcinomas, wherein the colon and colorectal carcinomas are drug-resistant.

The invention further relates to a method for the treatment of colon and colorectal carcinomas, or delaying the recurrence of colon and colorectal carcinomas, comprising the administration of and effective dose of ibrutinib and of an effective dose of fluorouracil.

The method according to the present invention advantageously allows for the treatment of colon and colorectal carcinomas, wherein the colon and colorectal carcinomas are drug-resistant.

In a first embodiment the method of treatment of a colon or colorectal cancer according to the present invention provides first administering to a patient an effective dose of ibrutinib and sequentially an effective dose of fluorouracil.

In accordance with the present invention the effective doses of ibrutinib and of fluorouracil can be provided in liquid, solid, and semisolid dosage forms depending on the routes of administration as indicated above.

The amount of ibrutinib and of the fluorouracil in the unit dosage form is determined by the dosage to be used on a patient in the methods of the present invention.

In a second embodiment the method comprises administering to a colon or colorectal cancer patient the effective doses of ibrutinib and of fluorouracil simultaneously.

In a further embodiment according to the present invention the effective doses of ibrutinib and of fluorouracil can be administered separately.

### EXAMPLES

### Example 1. In vitro cell proliferation assays

The *in vitro* proliferation of HCT116p53KO, SW480 and DLD-1 cells, three colon carcinoma cell lines, which are resistant to 5FU treatment, and the HCT116 cell line, which is sensitive to 5FU treatment, were grown and assayed up to 72h after seeding, in the presence of different concentrations of ibrutinib ranging from 0 to 10 µM.

As can be seen from the graphs of Figure 1, these short term assays indicate that ibrutinib does not alter the capacity of the cells to proliferate, even at a concentration of 10 µM.

### Example 2. In vitro colony assays

The HCT116p53KO, SW480, DLD-1 cells and HCT116 cell lines were seeded at low density and grown for 10-12 days in the presence of different concentrations of ibrutinib ranging from 0 to 20 µM.

As can be seen from the colony forming assays shown in Figure 2, these long term assays indicate that even in the long term, treatment with ibrutinib does not affect clonogenic and proliferative capabilities up to a concentration of 1µM. A concentration of 10 µM Ibrutinib decreases the number and the size of the colonies indicating that it affects both clonogenicity and proliferation, which are evntually inhibited at a concentration of 20 µM.

### Example 3. Effect of ibrutinib and fluorouracil treatment on cell viability

HCT116p53KO and HCT116 cell lines were grown *in vitro* for 72hs in the presence of different concentrations of Ibrutinib (from 0 to 20 µM) and of Ibrutinib (from 0 to 20 µM) + 5FU (200 µM )and their viability was evaluated at the end of the incubation using the calcein assay (a non fluorescent dye that become fluorescent upon cleavage by lysosomal esterases, active only in living cells) .

As can be seen from the graphs reported in Figure 3, concomitant administration of 200 µM 5FU (and ibrutinib re-sensitize resistant HCT116p53KO cells to the cytotoxic effect of 5FU starting from the concentration of 100nM, to achieve a maximal effect at 20 µM. In contrast, the addition of ibrutinib to 5FU in cell cultures of HCT116 (sensitive) has no additive effect on the response to chemotherapy.

### Example 4. Effect of ibrutinib on the response to targeted therapy

HCT116p53KO and HCT116 cell lines were grown *in vitro* and subjected to the combined treatment of ibrutinib and three different monoclonal antibodies currently used in anti-cancer therapy for targeting EGFR (cetuximab, panitumumab) and VEGF (bevacizumab), both in short term (upper panel) and in long term (lower panel) experiments

In the upper part of Figure 4 the graph shows that the combined treatment with 20 µM ibrutinib and these "targeted" drugs does not revert the resistance of HCT116p53KO cells, as assessed by Trypan blue staining 72hs after the combined treatment. As shown in the lower panel, also the continuous exposure for 10-12 days to the combined treatment does not re-sensitize resistant cells to targeted therapy.

### Example 5. In vivo treatment with ibrutinib and fluorouracil

Tumors were established by injecting s.c. 1 x10⁶ cells (in 100 µL of a 50% PBS and 50% Matrigel solution), HCT116p53KO cells into the left flanks and HCT116 into the right flanks, of 5 to 7 weeks old female CD-1 nude mice. When HCT116p53KO tumors reached the average volume of 100 mm³ (day 8 postengraftment), animals were randomized and given vehicle, 5FU [via intraperitoneal (i.p.) injection, 60 mg/kg, twice a week], Ibrutinib [via oral gavage, 25 mg/kg (mpk) or 50 mpk once day for 5 days a week], or a combination thereof. 5FU treatment started at day 9 post-engraftment, whereas Ibrutinib treatment started at day 8 post-engraftment. Control mice received i.p. injections of vehicle (0.9% NaCl solution) with the same schedule of the other groups. Tumors were measured with caliper once a week. Statistical significance was determined with a Kruskal- Wallis non parametric test (normal distribution not assumable), followed by Nemenyi-Damico-Wolfe-Dunn test for multiple pairwise comparisons between groups. In all cases, a P value < 0.05 was considered as significant

As can be seen from the graph reported in Figure 5a, after 28 days of treatment the average volume of the tumor masses formed by xenografted drug-resistant HCT116p53KO amounts to ≈955mm³ for the group of untreated mice (vehicle), to ≈760mm³ for the group treated with ibrutinib (25mpk) alone, to ≈530mm³ for the group treated with 5FU alone and to ≈315mm³ for the group treated with the combination of ibrutinib and 5FU. As illustrated in Figure 5b statistical analysis shows that the reduction in the tumor volume is not significant when comparing the group of mice treated with 5FU alone vs the untreated group but it become significant when comparing the group of mice treated with 5FU+Ibrutinib vs. the untreated group and vs. the group treated with Ibrutinib alone.

Data reported in Figure 6a show that doubling the dose of Ibrutinib (50 mpk) administered to mice do not further decrease the volume of the tumors of the group of mice treated with the combination 5FU+Ibrutinib (compared to the group treated with 5FU+Ibru 25 mpk). However, the treatment with the high dose of Ibrutinib has an anti-tumoral effect on its own: the statistical analysis reported in Figure 6b shows that, when compared to the untreated group, the reduction in the tumor volume obtained administering to the mice Ibrutinib 50 mpk is as significant as that obtained administering the combination 5FU+Ibrutinib 50 mpk.

These data show that the combined treatment with ibrutinib and fluorouracil has a synergistic effect on the tumor volume reduction when low doses of Ibrutinib are administered together with chemotherapy to mice bearing drug-resistant xenografts and that an higher dosage of Ibrutinib has an anti-tumoral effect on its own. The graphs reported in Figure 7 show the results of the analysis performed on the tumoral tissues excised from the mice at the end of the different treatments. To evaluate the proliferative index tissue sections were stained with antibodies against Ki67, a marker of proliferating cells. Percentage of Ki67-positive cells was quantified by Scanscope dedicated software upon digital acquisition of Ki67-stained, hematoxylin-eosin counter-stained slides (Figures 7a and b). Using another feature of the dedicated software also the percentage of necrotic area was calculated on the same slides (Figure 7c).

These data show that both, at low and high doses, neither Ibrutinib alone nor the combination Ibrutinib+5FU affect the proliferation of tumor cells whereas the combination increases significantly the extension of the necrotic area.

The graph in Figure 8a, represents the growth curve of tumors derived from HCT116 xenografted in CD1 mice after 28 days of treatment with vehicle alone, Ibrutinib (25mpk), 5FU (60mpk) and Ibrutinib (25mpk) + 5FU (60mpk). By the end of the treatment the average volume of the tumor masses formed by xenografted drug-sensitive HCT116 cells amounts to ≈435mm³ for the group of untreated mice (vehicle), to ≈347mm³ for the group treated with ibrutinib (25mpk) alone, to ≈226mm³ for the group treated with 5FU alone and to ≈172mm³ for the group treated with the combination of ibrutinib and 5FU. The difference in tumor size is not statistically significant between veichle-treated and ibrutinib-treated groups nor between 5FU-treated and 5FU+ibrutinib-treated groups. Reduction in the tumor volume is in fact significant only when comparing the group of mice treated with 5FU ± Ibrutinib vs the untreated (or Ibrutinib-treated) group.

Data plotted in the graph of Figure 8b represent the percentage of proliferating cells in the tumoral tissues excised from the mice at the end of the treatments shown above: 5FU treatment ± Ibrutinib significantly reduces the percentage of proliferating cells compared to either vehicle or Ibrutinib alone.

The graph in Figure 9a, represents the growth curve of tumors derived from HCT116 xenografted in CD1 mice after 28 days of treatment with vehicle alone, Ibrutinib (50mpk), 5FU (60mpk) and Ibrutinib (50mpk) + 5FU (60mpk). By the end of the treatment the average volume of the tumor masses formed by xenografted drug-sensitive HCT116 cells amounts to ≈435mm³ for the group of untreated mice (vehicle), to ≈372mm³ for the group treated with ibrutinib (50mpk) alone, to ≈226mm³ for the group treated with 5FU alone and to ≈223mm³ for the group treated with the combination of ibrutinib and 5FU. The difference in tumor size is not statistically significant between veichle-treated and ibrutinib-treated groups nor between 5FU-treated and 5FU+ibrutinib-treated groups. Reduction in the tumor volume is in fact significant only when comparing the group of mice treated with 5FU ± Ibrutinib vs the untreated (or Ibrutinib-treated) group.

Data plotted in the graph of Figure 9b represent the percentage of proliferating cells in the tumoral tissues excised from the mice at the end of the treatments shown above: 5FU treatment ± Ibrutinib significantly reduces the percentage of proliferating cells compared to either vehicle or Ibrutinib alone.

All together these data show that the growth-reducing and anti-proliferative effects of 5FU on sensitive xenografts are achieved independently from the addition of Ibrutinib, which by itself is ineffective in these kind of tumors.

### Example 6. Treatment with ibrutinib and fluorouracil: lower amounts of chemotherapeutic drug are sufficient

In Figure 10 is depicted the synergistic effect of singularly ineffective concentrations of 5FU and Ibrutinib in drug-resistant and -sensitive colon carcinoma cells. Figure 10a shows representative photographs of drug-resistant HCT116p53KO cells taken at 72 hours of incubation in presence of 200 µM 5FU, 20 µM Ibrutinib, 200 µM 5FU + 20 µM Ibrutinib: it is evident that only in the latter experimental condition few cells are still attached to the plate and that the vast majority of the cells are dead. Figure 10b shows representative photographs of sensitive HCT116 cells taken at 72 hours of incubation in presence of non-toxic concentrations of 5FU and Ibrutinib. When the two drugs are used singularly (10 µM 5FU and 50 µM Ibrutinib) no toxicity is evident, whereas when the two drugs are used in combination (10 µM 5FU + 50 µM Ibrutinib) all the cells are detached from the culture dish because are dead. The fourth picture, shown for comparison, is of a culture dish where HCT116 have been treated with the maximally effective concentration of 200 µM.

These data clearly show that combining singularly ineffective concentrations of 5FU and Ibrutinib not only sensitize drug-resistant cells to chemotherapy but also allows to reduce the concentration of 5FU necessary to kill sensitive cells.

From the above description and the above-noted examples, the advantage attained by the product described and obtained according to the present invention are apparent.

## Claims

1. Pharmaceutical kit for simultaneous, sequential and separate use of an effective dose of ibrutinib and an effective dose of fluorouracil, in the treatment of colon and colorectal carcinomas.

2. Pharmaceutical kit according to claim 1, wherein the colon and colorectal carcinomas are drug-resistant.

3. Pharmaceutical kit according to anyone of claims 1 or 2, wherein the ibrutinib is in the form of a pharmaceutically acceptable salt.

4. Pharmaceutical kit according to anyone of claims 1 to 3, wherein the effective dose of ibrutinib and the effective dose of fluorouracil are formulated as a pharmaceutical form selected from oral form, parenteral form and rectal form.

5. Pharmaceutical kit according to claim 4, wherein the pharmaceutical form is an oral form, preferably a tablet.

6. Pharmaceutical kit according to claim 4, wherein the pharmaceutical form comprises at least one pharmaceutical acceptable excipient.

7. Pharmaceutical kit according to anyone of claims 1 to 6, wherein the effective dose of ibrutinib is in the range of from 25 mg/day to 840 mg/day.

8. Pharmaceutical kit according to anyone of claims 1 to 7, wherein the effective dose of fluorouracil is in the range of from 400 mg/m² to 600 mg/m².

9. Pharmaceutical kit according to anyone of claims 1 to 8, comprising at least one container comprising the effective dose of ibrutinib and at least one container comprising the effective dose of fluorouracil, and an instruction leaflet.

10. Use of an effective dose of ibrutinib and an effective dose of fluorouracil, in the treatment of colon and colorectal carcinomas.

11. The use according to claim 10, wherein the colon and colorectal carcinomas are drug-resistant.

12. A method for the treatment colon and colorectal carcinomas, comprising the administration of and effective dose of ibrutinib and of an effective dose of fluorouracil.

13. The method according to claim 12, wherein the colon and colorectal carcinomas are drug-resistant.
